(19) Europäisches Patentamt
European Patent Office
Office européen des brevets

(11) Publication number: 0 303 437
A2

(12) # EUROPEAN PATENT APPLICATION

(21) Application number: 88307341.3

(22) Date of filing: 09.08.88

(51) Int. Cl.⁴: A 61 K 31/70

(30) Priority: 10.08.87 US 84523

(43) Date of publication of application:
15.02.89 Bulletin 89/07

(84) Designated Contracting States:
AT BE CH DE ES FR GB GR IT LI LU NL SE

(71) Applicant: ENGELHARD CORPORATION
Menlo Park, CN 40
Edison New Jersey 08818 (US)

(72) Inventor: Amundsen, Alan R.
12 Meadowbrook Drive
Somerville New Jersey 08876 (US)

Stern, Eric W.
234 Oak Tree Road
Mountainside New Jersey 07092 (US)

Hollis, Leslie Steven
33 Guilford Lane
Mercerville New Jersey 08619 (US)

(74) Representative: Fisher, Adrian John et al
CARPMAELS & RANSFORD 43 Bloomsbury Square
London WC1A 2RA (GB)

The title of the invention has been amended (Guidelines for Examination in the EPO, A-III, 7.3).

(54) **Platinum triamine compounds containing nucleotides or nucleosides as antitumor agents.**

(57) Compositions containing platinum triamine antitumor agents containing nucleotides or nucleosides are disclosed. A method of treating malignant animal tumor cells sensitive to such compounds and compositions is also disclosed.

EP 0 303 437 A2

**Description**

## COMPOSITIONS CONTAINING PLATINUM TRIAMINE ANTITUMOR AGENTS CONTAINING NUCLEOTIDES OR NUCLEOSIDES AND METHOD OF USING THE SAME

### Field of the Invention

The present invention relates to pharmaceutical compositions comprising at least one of certain platinum triamine antitumor complexes containing a nucleotide or nucleoside, in admixture with a pharmaceutically acceptable diluent or carrier. The invention also relates to the use of such a composition or complex in therapy, in particular for the treatment of malignant tumors in animals, especially in mammals.

### Background of the Invention

Cisplatin has proven effective in the treatment of a variety of forms of cancer such as testicular and ovarian carcinoma. A continuing effort has been put forth in analog development programs to broaden the spectrum of activity and to improve the therapeutic properties of platinum-based antitumor agents. These goals include developing agents that demonstrate activity in unresponsive diseases, such as colon, breast, stomach and brain cancer and improving on the activity seen in tumor systems that presently respond to cisplatin, such as lung, bladder, and head and neck carcinomas.

During the past fifteen years, several thousand platinum complexes have been prepared and evaluated in a variety of animal tumor screens in an attempt to devise structure activity-relationships among the platinum analogs. The classical structure-activity relationships were first summarized by Cleare and Hoeschele in Bioinorg. Chem. 1973, 2, 187, and since that time these guidelines have continued to dominate the search for new analogs. In general, platinum(II) complexes of the form cis-$PtA_2X_2$, wherein A is an amine ligand and X is anionic leaving group, represent the majority of the active analogs. (Prestayko, A.E. et al. "Cisplatin Current Status and New Developments", Academic Press, New York 1980 and Hacker, M.R. et al. "Platinum Coordinations Complexes in Cancer Chemotherapy," Martinus- Nijhoff, Boston, 1984). The corresponding trans-diamine complexes are devoid of activity in all cases. The importance of the cis geometry of the leaving groups has been stressed in many studies that suggest the mechanism of antitumor activity of these compounds is related to their ability to inhibit replication by binding to adjacent guanine bases on duplex DNA. Pinto et al, Biochem. Biophys. Acta. 1985, 780, 167, Johnson et al, in "Biochemical Mechanisms of Platinum Antitumor Drugs", IRL Press, Washington, D.C. 1986, pl, and Roberts et al, ibid., p. 29.

Aside from those complexes conforming to the basic cis-$PtA_2X_2$ structural class, relatively few platinum compounds have demonstrated activity in in vivo tumor systems.

This application discloses chemical and biological data on a series of active platinum(II) antitumor agents that violate some of the classical structure-activity relationships. The compounds disclosed in this application are platinum triamine complexes of the structure type shown below wherein $A_1$ and $A_2$ are the same or different and are selected from ammonia or a monodentate amine, or when taken together represent a bidentate amine, L is a nucleotide or nucleoside, X is an anionic ligand and Y is an anion. Usually $n = 1$. However, under certain conditions, L may be anionic in which case $n = 0$ and Y is not present.

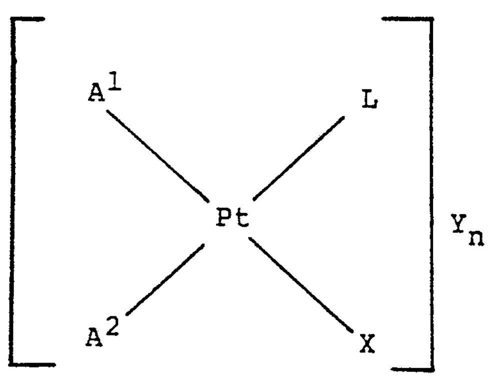

I

Members from this class of compounds have demonstrated activity in a number of in vivo antitumor screens, including S180a and L1210 systems. These platinum-triamine complexes, which may be cationic species at

neutral pH, also possess desirable physical properties, such as high stability and solubility in aqueous media.

Complexes identified as or suggested to be similar to those prepared during this work have been reported. Cationic triamine complexes containing one nucleoside per platinum are reported for: (1) cytidine, Stetsenko, A.I.; Alekseeva, G.M.; Yakovlev, K.I. Zh. Neorg. Khim. 1985, 30, 2592, Stetsenko, A.I.; Sel'derkhanova, L.B.; Mokhov, A.I. Koord. Khim. 1985, 11, 816, Mânsy, S.; Rosenberg, B.; Thomson, A.J. J. Amer. Chem. Soc. 1973, 95, 1633, Stetsenko, A.I.; Adamov, O.M.; Titov, V.E.; Dmitrieva, E.S.; Yakovlev, K.I. Koord. Khim. 1983, 9, 851, Nee, M.; Roberts, J.D. Biochemistry 1982, 21, 4920, Tang W.; Zhang, C.; Tai, A. Gaodeng Xuexiao Huaxue Xuebao 1981, 2, 423, (2) deoxycytidine, Inagaki, K.; Tamaoki, N.; Kidani, Y. Inorg. Chim. Acta 1980, 46, L93, (3) guanosine, Nee, M.; Roberts, J.D. Biochemistry 1982, 21, 4920, Tang, W.; Zhang, C.; Tai, A. Gaodeng Xuexiao Huaxue Xuebao 1981, 2, 423, Okamot, K.; Behnam, V.; Gauthier, J.Y.; Hanessian, S.; Theophanides, T. Inorg. Chim. Acta 1986, 123, L1., Pasini, A.; Calmotti, S. Congr. Naz. Chim. Inorg. [Atti], 16th, Univ. Studi Ferrara; Ferrara, Italy, 1983, p436, Goulotti, M.; Pacchioni, G.; Pasini, A.; Ugo, R. Inorg. Chem. 1982, 21, 2006, Peresie, H.J.; Kelman, A.D. Inorg. Chim. Acta 1978, 29, L247, Miller, S.K.; Marzilli, L.G. Inorg. Chem. 1985, 24, 2421, Ward, S.G.; Taylor, R.C. NATO ASI Ser., Ser C 1984, 124, 767, Scovell, W.M.; O'connor, T. J. Clin. Hematol. Oncol. 1977, 7, 487, (4) deoxyguanosine, Inagaki, K.; Tamaoki, N.; Kidani, Y. Inorg. Chim. Acta 1980, 46, L93, Kulamowicz, I.; Olinski, R.; Walter, Z. Z. Naturforsch, C: Biosci. 1984, 39C, 180 and (5) 1-β-D-arabinofuranosylcytosine, Neidle, S.; Taylor, G.L.; Robins, A.B. Acta Crystallogr. Sect B 1978, B34, 1838, Robins, A.B. Chem.-Biol. Interact. 1976, 13, 205. Additionally, cationic triamine species are cited for (1) adenosine, Mansy, S.; Rosenberg, B.; Thomson, A.J. J. Amer. Chem. Soc. 1973, 95, 1633, Scovell, W.M.; O'connor, T. J. Clin. Hematol. Oncol. 1977, 7, 487., and (2) deoxyadenosine, Inagaki, K.; Tamaoki, N.; Kidani, Y. Inorg. Chim. Acta 1980, 46, L93. Neutral triamine species are reported for (1) guanosine, Pasini, A.; Mena, R. Inorg. Chim. Acta 1981, 56, L17, (2) uridine, Lim, M.C.; Martin, R.B. J. Inorg. Nucl. Chem. 1976, 38, 1915 and (3) thymidine, Lim, M.C.; Martin, R.B.; J. Inorg. Nucl. Chem. 1976, 38, 1915, in which the nucleosides are deprotonated. Complexes of 1-methylguanosine, 7-methylguanosine, and 1-methyladenosine, (O'connor, T.; Scovell, W.M. Chem.-Biol. Interact. 1979, 26, 227), have also been proposed.

In few, if any, of the above cases have pure materials actually been isolated, although isolation is claimed in the case of (1) cytidine, Stetsenko, A.I.; Alekseeva, G.M.; Yakovlev, K.I. Zh. Neorg. Khim. 1985, 30, 2592., Stetsenko, A.I.; Sel'derkhanova, L.B.; Mokhov, A.I. Koord. Khim. 1985, 11, 816, Stetsenko, A.I.; Adamov, O.M.; Titov, V.E.; Dmitrieva, E.S.; Yakovlev, K.I. Koord. Khim 1983, 9, 851, (2) guanosine, Peresie, H.J.; Kelman, A.D. Inorg. Chim. Acta 1978, 29, L247, Kulamowicz, I.; Olinski, R.; Walter, Z. Z. Naturforsch, C: Biosci. 1984, 39C, 180, (3) adenosine, Stetsenko, A.I.; Adamov, O.M.; Titov, V.E.; Dmitrieva, E.S.; Yakovlev, K.1. Koord. Khim 1983, 9, 851 and (4) 1-β-D-arabinofuranosylcytosine, Neidle, S.; Taylor, G.L.; Robins, A.B. Acta Crystallogr. Sect. B 1978, B34, 1838, Robins, A.B. Chem.-Biol. Interact. 1976, 13, 205. In the case of arabinofuranosylcytosine, a single crystal X-ray structure was determined.

Triamine complexes containing 1-methylcytosine, (Lippert B.; Lock, C.J.L.; Speranzini, R.A. Inorg. Chem. 1981, 20, 335, Faggiani, R.; Lock, C.J.L.; Lippert, B. Inorg. Chim. Acta 1985, 106, 75), and 9-ethylguanine, (Raudaschl-Sieber, G.; Marzilli, L.G.; Lippert, B.; Shinozuka, K. Inorg. Chem. 1985, 24, 989), in which the alkyl substituted nucleobase constitutes a model for the corresponding nucleoside, have also been structurally characterized.

A significant body of published work deals with the interaction of cisplatin with nucleobases, nucleosides, nucleotides, dinucleotides, oligonucleotides and DNA. The objective of this work is the elucidation of the exact nature of the cisplatin/DNA lesion and not the preparation of new antitumor agents, (Sherman, S.E.; Gibson, D.; Wang, A.H.J.; Lippard, S.J. Science 1985, 230, 412.)

None of the prior art, however, discloses the use of a nucleoside containing platinum triamine as an anticancer agent. Activity in other compounds has been reported. One report finds activity present in a series of complexes of the type $PtACl_2$, wherein A is a chelating amine prepared by amination of cytidine, deoxycytidine and 1-β-D-arabinofuranosylcytosine, Maeda, M.; Abiko, N.; Uchida, H.; Sasaki, T. J. Med. Chem. 1984, 27, 444, Maeda, M.; Abiko, N.; Uchida, H.; Sasaki, T. Nucleic Acids Symp. Ser. 1982, 11, 131. Another report cites activity for the tetramine species $[PtLL'_2]Cl_2$ where $L$ = a chelating diamine and $L'$ = guanosine, Ugo, R.; Gulotti, M.; Pasini, A. Congr. Naz. Chim. Inorg., [Atti], 13th, 1980, p84. Two other reports claim that cisplatin loses its antimitotic activity against cells in culture when bound to guanosine, (Maeda, M.; Abiko, N.; Uchida, H.; Sasaki, T. J. Med. Chem. 1984, 27, 444, Heinen, E.; Bassleer, R.; Desaive, C. Eur. J. Cancer 1978, 14, 1005), and other purine nucleosides, (Maeda, M.; Abiko, N.; Uchida, H. Sasaki, T. J. Med. Chem. 1984, 27, 444). Several of the platinum pyrimidine "blues" have also shown antitumor activity, Lippert, B. J. Clin. Hematol. Oncol. 1977, 7, 26.

U.S. patent application Serial No. 723,783 filed on April 16, 1985 naming the same inventors as named herein discloses a series of platinum triamine complexes that exhibit antitumor activity. Similarly, U.S. patent application Serial No. 07/077,269, filed on July 24, 1987 naming the same inventors as named herein discloses platinum triamine antitumor agents containing saturated amines that exhibit antitumor activity. These US applications correspond, respectively to EP-A-0,199,524 and European Patent Application No. 88306758.9.

## Detailed Description of the Invention

The compounds of this invention are represented by the following general formula:

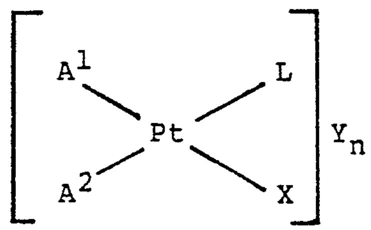

In this structure $A^1$ and $A^2$ are the same or different and are a monodentate amine, such as $R^1NH_2$ wherein $R^1$ is a hydrogen, or linear or branched $C_1$-$C_6$ alkyl group which may bear one or more substituents selected from hydroxy,$C_1$-$C_6$ alkyl, $C_1$-$C_4$ alkyl, halo, carboxy, $C_1$-$C_4$ alkylcarboxy or $C_1$-$C_4$ alkoxycarbonyl. $A^1$ and $A^2$ when taken together are a diamine of the formula:

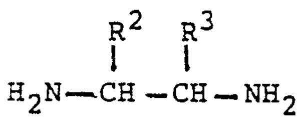

wherein $R^2$ and $R^3$, taken separately are a linear or branched $C_1$-$C_6$ alkyl group which may bear one or more substituents as defined above in connection with $R^1$. $R^2$ and $R^3$ taken together form a 1,2-diaminocycloalkane containing 4-8 nuclear carbon atoms which may be substituted on the carbons by one or more of the substituents selected from hydroxy,$C_1$-$C_6$, alkyl, $C_1$-$C_4$ alkoxy, halo, carboxy, $C_1$-$C_4$ alkylcarboxy or $C_1$-$C_4$ alkoxycarbonyl.

L is a purine or pyrimidine nucleoside or nucleotide which may also bear on the purine or pyrimidine nucleus one or more substituents selected from $C_1$-$C_6$ alkyl, $C_1$-$C_6$ alkoxy, hydroxy, carboxy, $C_1$-$C_6$ alkoxycarbonyl, $C_1$-$C_6$ alkylcarboxy, amino, oxo, nitro or amido. As defined herein a purine or pyrimidine nucleoside or nucleotide includes both the neutral species and an anionic species since under certain conditions, especially certain pH conditions, L may be anionic.

The presently preferred L species are guanosine represented by the formula:

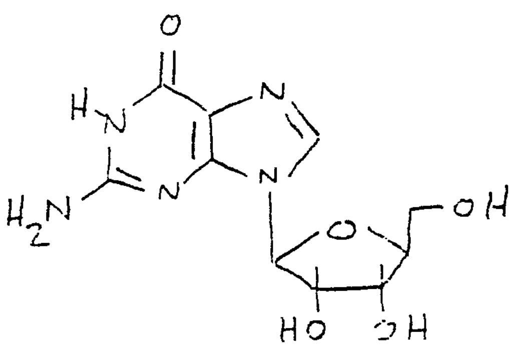

2′-deoxyguanosine represented by the formula:

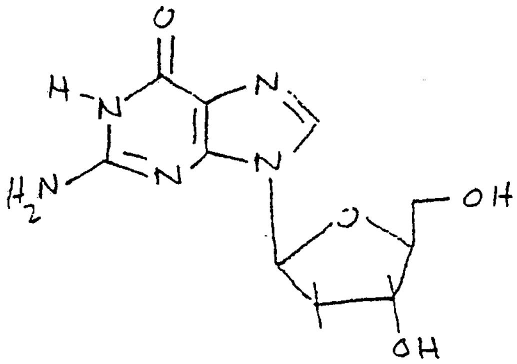

cytidine represented by the formula:

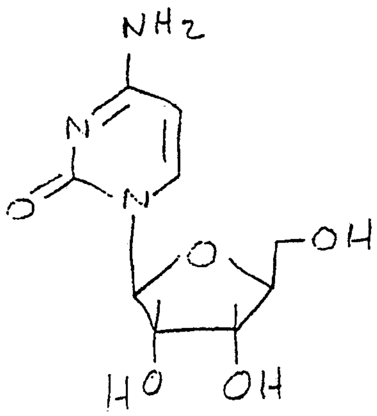

2′deoxycytidine represented by the formula:

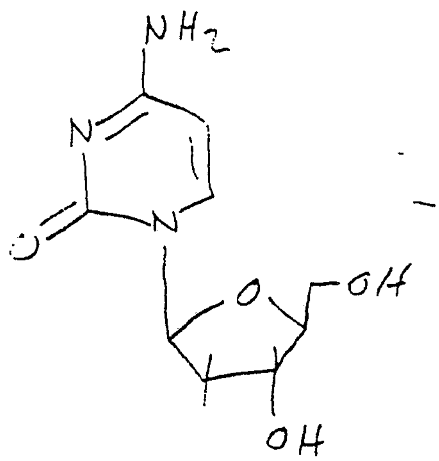

and 1-β-D-arabinofuranosylcytosine represented by the formula:

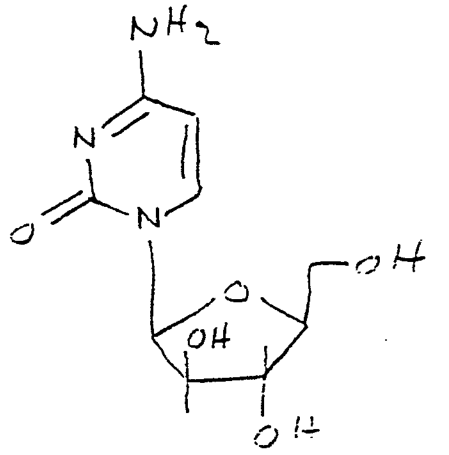

X is a monodenate anionic ligand such as halo, hydroxo, pseudohalo, $HSO_4$, $H_2PO_4$, ascorbate, or $C_1$-$C_6$ carboxylate.
Y is an anion chosen from X above or nitrate or perchlorate. When, however, L is anionic Y is not present. Thus n = 0 or 1. X may or may not be the same as Y.

## Pharmacology

The products of this invention are useful in the treatment of malignant tumor cells sensitive thereto in animals as, for example, Sarcoma 180 ascites and L1210 leukemia in mammals such as mice. This antitumor cell effect also may extend to other sarcomas and leukemias and to such other tumor cells as lymphoid leukemia, lymphosarcoma, myelocytic leukemia, malignant lymphoma, squamous cell carcinoma, adenocarcinoma, scirrhous carcinoma, malignant melanoma, seminoma, teratoma, choriocarcinoma, embryonal carcinoma, cystadenocarcinoma, endometroidcarcinoma or neuroblastoma and the like. In addition, the complexes may be useful as anti-viral, anti-inflammatory, anti-bacterial and anti-parasitic agents.

The complexes of this invention may be administered parenterally or orally in a mixture with a non-toxic pharmacologically acceptable inert carrier or diluent in any of the usual pharmaceutical forms. These include solid and liquid oral unit dosage forms such as tablets, capsules, powders and suspensions or solutions and suspensions for subcutaneous, intramuscular, intravenous, or intraarterial injection.

The term "unit dosage" refers to physically discrete units which may be administered in a single or multiple dosages each containing a predetermined quantity of active ingredient in association with the required diluent, carrier or vehicle. The quantity of active ingredient is the amount of the complex which is needed to produce the desired therapeutic effect.

A typical unit dosage consists essentially of from about 10 to 450 mg of active ingredient; however, the form in which the ingredient is administered and the frequency of administration is usually determinative of the concentration. Thus, for example, oral unit dosage forms containing 20 to 450 mg of active ingredient may be administered one or more times per day depending upon the severity of the tumor cells which is sought to be treated and the condition of the host animal. By contrast, parenteral administration generally requires from about 10 to about 100 mg of the active ingredient per unit dosage administered as a daily dose or as a fraction thereof depending upon whether the regimen calls for administration once, twice, three or four times daily.

By contrast to the unit dosage, the effective dose is that dosage which is needed to achieve the desired anti-tumor effect. In general, this dosage lies within the range of from about 10 to 950 mg of the active ingredient per kg of body weight of the host animal. A preferred concentration lies within the range of from about 30 to 450 mg/kg of body weight. For oral administration it has been found that an effective dose of about 50 to 950 mg/kg is most suitable, whereas in the case of parenteral administration it is usually advisable to employ from about 30 to 350 mg/kg. These unit dosages are well below the toxic or lethal dose and they may be varied over a wide range for adjustment to the patient who is being treated.

According to the present invention, the term "pharmacologically acceptable inert carrier or diluent" means a non-toxic substance which, when mixed with the active ingredient, renders it more suitable for administration. Compositions intended for oral administration may include such carriers or diluents as corn starch, potato starch, sodium carboxymethyl cellulose, ethyl cellulose, cellulose acetate, powder gum tragacanth, gelatin, alginic acid, agar, stearic acid or the sodium, calcium and magnesium salts of stearic acid, sodium lauryl sulfate, polyvinylpyrrolidone, sodium citrate, calcium carbonate and dicalciumphosphate. The compositions may also contain non-toxic adjuvants and modifiers such as dyes, buffering agents, preservatives, surfactants, emulsifiers, flavoring agents, biocides and the like.

Tablets are prepared by mixing a complex of this invention in a suitably comminuted or powdered form with a diluent or base such as starch, kaolin, dicalciumphosphate and the like. The resulting mixture can be granulated by wetting with a binder such as a syrup, starch (paste), acacia mucilage or solutions of cellulosic or polymeric materials, whereafter, the wetted mixture is formed through a screen. As an alternative to granulating, the powder mixture can be run through a tablet machine and any imperfectly formed slugs broken into granules. The granules are lubricated to prevent sticking to the tablet-forming dyes via the addition of stearic acid, a stearate salt, talc or mineral oil and the lubricated mixture is then compressed into tablets. The complexes can also be combined with free flowing inert carriers followed by compression into tablets without going through the granulated or slugging steps. A protective coating or sealing coat of shellac, sugar or polymeric material and a polished coating of wax can also be provided. Dye stuffs may be added to distinguish different unit dosages.

Dry or hard filled capsules are formulated by preparing a powdered mixture, according to the procedure herein before described and pouring the mixture into preformed gelatin sheets. A lubricant such as talc, magnesium stearate or calcium stearate can be added prior to the filling operation. A glidant such as colloidal silica may be added to improve the flow characteristics and a disintegrating or solubilizing agent may also be added to enhance the effectiveness of the medicament upon ingestion.

In soft gelatin capsules, the active complex is dissolved or suspended in vegetable oil, peanut oil, alcohol or glycerine and the like.

Powders for addition to foods, drinking water, fruit juice or other potable liquids are prepared by comminuting the compound to a fine size and mixing with a similarly comminuted pharmaceutical diluent or carrier such as an edible carbohydrate as, for example, starch. Sweetening agents and flavorings, preservatives and dispersing and/or coloring agents may also be employed.

Oral fluids such as syrups and elixirs are prepared in unit dosage forms so that a given quantity of medicament, such as a teaspoonful, will contain a predetermined amount of the active ingredient. Suspensions can be formulated by dispersing the active ingredient in a non-toxic vehicle in which it is essentially insoluble.

Compositions intended for parenteral administration may include such diluents and carriers as water or water-miscible solvents as, for example, sesame oil, groundnut oil, aqueous propylene glycol and a solution of sodium riboflavin. Typical of said compositions are solutions which contain the active ingredient in sterile form. Alternatively, a unit dosage form for parenteral administration can be prepared by placing a measured amount of complex in solution in sterile form, removing the solvent by lyophilization and sealing the vial. An accompanying vial of sterile vehicle can be provided for mixing with the complex prior to administration.

According to the present invention one or more of the complexes may be combined into a single unit dosage form or, alternatively, one or more of the complexes of the present invention may be combined with other known anti-tumor agents, therapeutic agents or nutrative agents so as to enhance or compliment the antitumor effect.

The preferred compositions for oral administration are tablets in which the complexes of the present invention are present in quantities of about 5 to about 375 mg but, preferably, about 10 to 200 mg in a pharmaceutically acceptable orally ingestible solid carrier. If desired, the compositions may also contain flavorants, binders, lubricants and other excipients known in the art.

A preferred alternative for oral administration is the soft gelatin capsule. Such a composition may contain from about 5 to about 375 mg, but, preferably, about 10 to 200 mg by weight of active ingredient dissolved or suspended in vegetable oil, peanut oil, alcohol or glycerin and the like.

A preferred unit dosage form for parenteral administration will contain complexes of the present invention of about 10 to 100 mg, but preferably 15 to 75 mg.

The following embodiments illustrate representative unit dosage forms:

## Compressed Tablet

| | |
|---|---|
| cis-[Pt($NH_3$)$_2$(2'-deoxycytidine)Cl]Cl | 150 mg. |
| Niacinamide | 50 mg. |
| Calcium Pantothenate | 20 mg. |
| Magnesium Sulfate | 50 mg. |
| Zinc Sulfate | 50 mg. |
| Magnesium Stearate | 10 mg. |
| | 330 mg. |

The cis-[Pt($NH_3$)$_2$(2′-deoxycytidine)Cl]Cl complex, niacinamide, calcium pantothenate, magnesium sulfate, zinc sulfate and magnesium stearate (5.0 mg.) are mixed and compressed into slugs. The slugs are then broken into granules and sifted through an 8 mesh screen. Additional magnesium stearate (5.0 mg.) is added and the mixture is then compressed into tablets suitable for oral administration.

## Soft Gelatin Capsule

A soft elastic gelatin capsule is filled with the following ingredients:

| | |
|---|---|
| cis-[Pt($NH_3$)$_2$(guanosine)Cl]$NO_3$ | 200 mg. |
| Wheat germ oil | 50 mg. |
| Sunflower seed oil | 100 mg. |
| | 350 mg. |

The cis-[Pt($NH_3$)$_2$(guanosine)Cl]$NO_3$ complex and wheat germ oil are mixed with sunflower seed oil and the resulting mixture is poured into gelatin capsules suitable for oral administration. An alternative embodiment provides for substituting sunflower seed oil and wheat germ oil with equal amounts of peanut oil to obtain an otherwise similar capsule which is also suitable for oral administration.

## Dry Filled Capsule

A hard dry=filled capsule may be preparted from the following ingredients:

| | |
|---|---|
| cis-[Pt($NH_3$)$_2$(2'-deoxyguanosine)Cl]Cl | 250 mg. |
| Niacinamide | 50 mg. |
| Calcium Pantothenate | 10 mg. |
| Sodium Ascorbate | 150 mg. |
| | 460 mg. |

The cis-[Pt($NH_3$)$_2$(2'-deoxyguanosine)Cl]Cl complex is reduced to a No. 60 powder. Niacinamide, calcium pantothenate and sodium ascorbate are passed through a No. 60 bolting cloth and these ingredients are added to the said complex. This combination of ingredients is mixed for 10 minutes and then poured into a No. 3 size gelatin capsule.

## Dry Powder

The following composition illustrates a representative dosage in dry powder form. In this embodiment the active ingredient is water soluble and it is combined with up to 60% by weight of a suitable flavoring agent. All quantities are in a weight-percent relationship.

cis-[Pt($NH_3$)$_2$(1-β-D-arabinofuranosylcytosine 25-90%
Flavoring Agent 10-60%
Preservative 0.1%

## Parenteral Solution

Injectable solutions can be formulated by mixing an ampoule of active ingredient with an ampoule of sterile diluent:

cis-[Pt($NH_3$)$_2$(guanosine)Cl]$NO_3$ 100 mg.
Ampoule: Sterile water (Diluent for Injection) 5 cc.

The cis-[Pt($NH_3$)$_2$(guanosine)Cl]$NO_3$ complex and water are mixed thoroughly immediately prior to administration. If desired, one or more other active ingredients may be added to provide an injectable solution having enhanced therapeutic activity.

## Method of Preparation

The compounds of this invention may be prepared in either of two ways: (1) The complex cis-$PtA_2Cl_2$, and an equimolar amount of the ligand L are stirred in water for 8-72 hr (24-48 hr preferred) at 30-80 °C (50-60 °C preferred), and (2) cis-$PtA_2Cl_2$ is reacted with an equimolar amount of $AgNO_3$ in DMF for 6-72 hr (24-48 hr preferred) at 0-50 °C (20-30 °C preferred). The AgCl precipitate formed in method (2) is removed by filtration and an equimolar amount of L added to the filtrate. This is reacted for 8-48 hr (16-24 hr preferred) at 0-50 °C (20-30 °C preferred). In both methods the solvent is then removed under vacuum. The materials are purified by shaking the residue with $CH_2Cl_2$ to remove excess L (and DMF in method (2)), and recrystallization from hot water. Contamination with cis-$PtA_2Cl_2$ can be removed by first dissolving the product in a minimum of water (at 20-30 °C) and gradually adding absolute ethanol. The cis-$PtA_2Cl_2$ impurity (yellow) is precipitated first and removed by filtration. Further addition of ethanol followed by removal of ethanol/water azeotrope under vacuum produces a precipitate of purified product.

Examples 1-6 illustrate the method by which the compounds of this invention may be prepared. Example 7 describes the protocol used to evaluate efficacy in mice. These examples are illustrative only and the invention should not be construed as being limited thereto because it will be apparent to one of ordinary skill that obvious modifications may be effected and functionally equivalent reagents may be substituted for those recited without departing from the spirit or scope of this invention.

The compounds of the Examples are characterized by $^{195}Pt$ NMR (Table I), $^{13}C$ NMR (Table II), and by elemental analysis. HPLC was used to determine that the products were free of contamination by cis-$PtA_2Cl_2$, tetramine (cis-$[PtA_2L_2]X_2$) and free ligand L.

## EXAMPLE 1

cis-$[Pt(NH_3)_2Cl_2]$

In the present invention, the cis-$[Pt(NH_3)_2Cl_2]$ starting material was prepared from $K_2[PtCl_4]$ using the method of Dhara as described in Dhara, S.G.; Indian J. Chem, 1970, 8, 193 which is incorporated herein by reference.

## EXAMPLE 2

cis-$[Pt(NH_3)_2(cytidine)Cl]NO_3$

Cisplatin (6.0 g) and $AgNO_3$ (3.39 g) were stirred in 100 mL DMF at room temperature for 24 hr. The AgCl precipitate was removed by filtration, cytidine (4.86 g) was added and the mixture stirred for another 24 hr. The DMF was removed under vacuum, and the residue stirred with 150 mL $CH_2Cl_2$. The resulting solid was dissolved in 20 mL hot water and cooled. Yellow solid was removed by filtration, and ethanol added to the filtrate producing a white solid. This was recrystallized from 10 mL hot water; the yield was 2.00 g. Anal. Calc. for $PtC_9H_{19}N_6O_8Cl$: C, 18.97; H, 3.36; N, 14.75. Found: C, 19.26; H, 3.66; N, 14.73.

## EXAMPLE 3

cis-$[Pt(NH_3)_2(guanosine)Cl]NO_3$

Cisplatin (6.0 g) and $AgNO_3$ (3.39 g) were stirred in 100 mL DMF at room temperature for 24 hr. Silver chloride was removed by filtration and guanosine (5.66 g) was added, and the mixture stirred for an additional 24 hr. The DMF was removed under vacuum, and the residue shaken with 150 mL $CH_2Cl_2$ for 1 hr. The resulting solid was filtered, air dried, and recrystallized from 50 mL hot water (yield, 4.88 g). Anal. Calc. for $PtC_{10}H_{19}N_8O_8Cl$: C, 19.69; H, 3.14; N, 18.37. Found: C, 19.47; H, 3.48; N, 18.19.

## EXAMPLE 4

cis-[Pt($NH_3$)$_2$(2′-deoxycytidine)Cl[Cl.

Cisplatin (3.0 g) and 2′-deoxycytidine (2.27 g) were stirred in 500 mL water at 60 °C for 16 hr. Upon cooling, unreacted cisplatin was filtered off and the volume reduced to 50 mL. After another filtration the solution was taken to dryness and extracted with 200 mL hot methanol, filtered while hot, and refrigerated overnight. The resulting solid was collected and dried; the yield was 1.41 g. Anal. Calc. for $PtC_9H_{20}N_5O_5Cl_2$: C, 19.86; H, 3.70; N, 12.87. Found: C, 19.91; H, 3.92; N, 12.71.

## EXAMPLE 5

cis-[Pt($NH_3$)$_2$(1-β-D-arabinofuranosylcytosine)Cl]$NO_3$

Cisplatin (3.0 g) and $AgNO_3$ (1.69 g) were stirred in 50 mL DMF for 24 hr. After filtering, 1-β-D-arabinofuranosylcytosine (1.11 g) was added and stirring resumed for an additional 24 hr. The mixture was taken to low volume under vacuum, and shaken with 150 mL $CH_2Cl_2$ for 2 hr. A solid was filtered off and dissolved in 10 mL water. The solution was filtered and ethanol was added to precipitate a white solid (yield, 0.63 g). Anal. Calc. for $PtC_9H_{19}N_6O_8Cl$: C, 18.97; H, 3.36; N, 14.75. Found: C, 19.64; H, 4.01; N, 14.70.

## EXAMPLE 6

cis-[Pt($NH_3$)$_2$(2′-deoxyguanosine)Cl]$NO_3$

Cisplatin (1.01 g) and $AgNO_3$ (0.571 g) were stirred in 25 mL DMF for 24 hr. AgCl was filtered off, and 2′-deoxyguanosine (0.90 g) added, and the mixture stirred for another 24 hr. The DMF was removed under vacuum; 100 mL $CH_2Cl_2$ was added and the mixture shaken for 2 hr. The resulting solid was recrystallized once from hot water. It was then added to 5 mL cold water, the undissolved cisplatin was removed filtration, and the product recovered by removing the water as an azeotrope with ethanol (yield, 0.28 g). Anal. Calc. for $PtC_{10}H_{19}N_8O_7Cl$: C, 20.23; H, 3.23; N, 18.87. Found: C, 20.68; H, 3.79; N, 18.85.

TABLE I

$^{195}$Pt NMR Data for Nucleoside Containing Pt Triamines

| Compound | Chemical Shift[a] |
|---|---|
| cis-[Pt($NH_3$)$_2$(guanosine)Cl]$NO_3$ | -2290 |
| cis-[Pt($NH_3$)$_2$(2'-deoxyguanosine)Cl]Cl | -2280 |
| cis-[Pt($NH_3$)$_2$(cytidine)Cl]$NO_3$ | -2358 |
| cis-[Pt($NH_3$)$_2$(2'-deoxycytidine)Cl]Cl | -2357 |
| cis-[Pt($NH_3$)$_2$(1-$\beta$-D-arabinofuranosylcytosine) | -2355 |

[a] ppm vs $H_2PtCl_6$ at 0 ppm.

TABLE II

$^{13}C$ NMR Data For Nucleoside Containing Pt Triamines

| Compound | Chem. Shift[a] | Assignment |
| --- | --- | --- |
| cis-[Pt($NH_3$)2(guanosine)Cl]$NO_3$ | 156.9,154.4 | C2,C4 |
| | 113.4 | C5 |
| | 150.5 | C6 |
| | 139.6 | C8 |
| | 86.3 | C1' |
| | 73.7 | C2' |
| | 70.4 | C3' |
| | 85.2 | C4' |
| | 61.4 | C5' |
| cis-[Pt($NH_3$)$_2$(2'-deoxyguanosine)Cl]Cl | 157.0,154.4 | C2,C4 |
| | 114.3 | C5 |
| | 150.4 | C6 |
| | 140.0 | C8 |
| | 87.6 | C1' |
| | 39.2 | C2' |
| | 70.8 | C3' |
| | 84.8 | C4' |
| | 61.4 | C5' |
| cis-[Pt($NH_3$)$_2$(cytidine)Cl]$NO_3$ | 165.1,165.2 | C2 |
| | 154.1 | C4 |
| | 95.2,95.2 | C5 |
| | 141.6,142.0 | C6 |
| | 91.2,91.3 | C1' |
| | 74.2,74.3 | C2' |
| | 69.1,69.3 | C3' |
| | 83.9,84.2 | C4' |
| | 60.6,60.7 | C5' |
| cis-[Pt($NH_3$)$_2$(2'-deoxycytidine)Cl]Cl | 164.6 | C2 |
| | 153.7 | C4 |
| | 94.5 | C5 |
| | 141.3,141.6 | C6 |
| | 86.5,86.9 | C1' |
| | 38.8,38.9 | C2' |
| | 69.9 | C3' |
| | 86.6 | C4' |
| | 94.5 | C5' |

TABLE II Continued

$^{13}C$ NMR Data For Nucleoside Containing Pt Triamines

| Compound | Chem. Shift[a] | Assignment |
|---|---|---|
| cis-[Pt($NH_3$)$_2$-(1-β-D-arabinofuranosylcytosine) | 165.1,165.2 | C2 |
| | 154.0 | C4 |
| | 94.4 | C5 |
| | 142.8,142.8 | C6 |
| | 86.8,86.9 | C1' |
| | 75.2,75.3,75.5,75.5 | C2',C3' |
| | 83.6,83.8 | C4' |
| | 60.7,60.8 | C5' |

[a] ppm vs TMS at 0 ppm.

## EXAMPLE 7

### Antitumor Evaluation

The compounds of Examples 2-6 were tested for antitumor activity using both the Sarcoma 180 Ascites and the L1210 Leukemia tumor models. Each of the compounds was evaluated in the Sarcoma 180 Ascites screen and at least some of the compounds were also evaluated in the L1210 Leukemia tumor models.

### L1210 Screening Methodology

L1210 screening was conducted according to a published method. (Rose et al, Cancer Treat. Rep. 1982, 66, 135 which is incorporated herein by reference). Female $CDF_1$ mice (16 - 22 g) were implanted with 1 x $10^6$ tumor cells ip on day zero. Compounds were administered in 0.5 mL water ip on day one. Groups of 6 mice were used for each dose. A control group of mice received only tumor and 0.5 mL water. A positive control group received tumor and 8 mg/kg cisplatin in 0.5 mL 0.15 M NaCl. The test was terminated after three times the mean survival time (MST) of the control group, surviving mice were counted as dying on that day. Activity was determined based on the percent increase in MST of test mice over controls (%ILS). An ILS of ≧ 25% represents activity. The tumor line was maintained through weekly transfer of 5 x $10^4$ cells in DBA/2 mice.

### Sarcoma 180 Ascites Screening Methodology

Female CFW mice (18 - 25 g) were implanted with 2 x $10^6$ cells ip on day zero. Compounds were administered in 0.5 mL water ip on day one. Groups of 6 mice were used for each test dose as well as controls as defined above. The test was run for twice the MST of the control group, with survivors counted as dying on that day. An ILS of ≧ 50% indicates activity. The tumor line was maintained through weekly transfer of 4 x $10^6$ cells in CFW mice.

The results of the screening tests are reported in Tables III and IV.

TABLE III

S180a Antitumor Screening Data for Platinum Nucleoside Complexes

| Compound (vehicle) | Dose mg/kg | %ILS | Surv | Positive Control %ILS | Positive Control Surv |
|---|---|---|---|---|---|
| cis-[Pt($NH_3$)$_2$(guanosine)Cl]$NO_3$ (water) | 10 | 2 | 0/6 | 59 | 0/6 |
| | 20 | 15 | 1/6 | | |
| | 40 | 23 | 0/6 | | |
| | 80 | 16 | 1/6 | | |
| | 160 | 79 | 3/6 | | |
| | 320 | -45 | 1/6 | | |
| cis-[Pt($NH_3$)$_2$(2'-deoxyguanosine)Cl]Cl (water) | 10 | 2 | 0/6 | 86 | 4/6 |
| | 20 | 15 | 1/6 | | |
| | 40 | 76 | 4/6 | | |
| | 80 | 88 | 4/6 | | |
| | 160 | 85 | 4/6 | | |
| | 320 | 6 | 2/6 | | |
| cis-[Pt($NH_3$)$_2$(cytidine)Cl]$NO_3$ (water) | 10 | 6 | 0/6 | 54 | 0/6 |
| | 20 | 1 | 0/6 | | |
| | 40 | -4 | 0/6 | | |
| | 80 | -7 | 0/6 | | |
| | 160 | 8 | 0/6 | | |
| | 320 | 30 | 0/6 | | |
| cis-[Pt($NH_3$)$_2$(2'-deoxycytidine)Cl]Cl (water) | 10 | 10 | 0/6 | 86 | 4/6 |
| | 20 | 34 | 0/6 | | |
| | 40 | 61 | 2/6 | | |
| | 80 | 85 | 3/6 | | |
| | 160 | 76 | 4/6 | | |
| | 320 | 51 | 1/6 | | |
| cis-[Pt($NH_3$)$_2$(1-β-D-arabinofuranosyl-cytosine)Cl]$NO_3$ (water) | 10 | -12 | 0/6 | 82 | 3/6 |
| | 20 | -3 | 0/6 | | |
| | 40 | 2 | 0/6 | | |
| | 80 | 42 | 2/6 | | |
| | 160 | 51 | 1/6 | | |
| | 320 | 45 | 2/6 | | |

TABLE IV

L1210 Antitumor Screening Data For Platinum Nucleoside Complexes

| Compound (vehicle) | Dose mg/kg | %ILS | Surv | Positive Control %ILS | Positive Control Surv |
|---|---|---|---|---|---|
| cis-[Pt($NH_3$)$_2$(guanosine)Cl]$NO_3$ (water) | 10 | -24 | 0/6 | 64 | 0/6 |
| | 20 | -4 | 0/6 | | |
| | 40 | -4 | 0/6 | | |
| | 80 | 2 | 0/6 | | |
| | 160 | 8 | 0/6 | | |
| | 320 | -26 | 0/6 | | |
| cis-[Pt($NH_3$)$_2$(2'-deoxycytidine)Cl]$NO_3$ (water) | 10 | 12 | 0/6 | 115 | 0/6 |
| | 20 | 23 | 0/6 | | |
| | 40 | 26 | 0/6 | | |
| | 80 | 44 | 0/6 | | |
| | 160 | 41 | 0/6 | | |
| | 320 | 59 | 0/6 | | |
| cis-[Pt($NH_3$)$_2$(Ara-C)Cl]$NO_3$ (water) | 10 | -22 | 0/6 | 48 | 0/6 |
| | 20 | -8 | 0/6 | | |
| | 40 | 0 | 0/6 | | |
| | 80 | 6 | 0/6 | | |
| | 160 | 10 | 0/6 | | |
| | 320 | 10 | 0/6 | | |

**Claims**

1. A composition for treating malignant animal tumors sensitive thereto comprising an active ingredient in admixture with a non-toxic pharmacologically acceptable inert diluent or carrier, characterised in that the active ingredient comprises a compound of the general formula I:

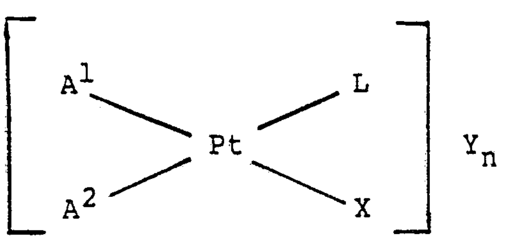

wherein Pt is in a valence state II and is coordinated to $A^1$ and $A^2$ in a cis configuration,
wherein $A^1$ and $A^2$ are the same or different and are each selected from monodentate amines and substituted monodentate amines, or $A^1$ and $A^2$ taken together are a diamine represented by the general formula:

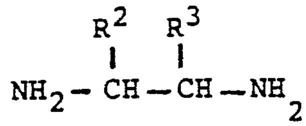

wherein $R^2$ and $R^3$ are the same or different and are each selected from linear or branched $C_1$-$C_6$ alkyl groups and substituted linear or branched $C_1$-$C_6$ alkyl groups, or $R^2$ and $R^3$, taken together, form a 1,2-diaminocycloalkane containing 4-8 nuclear carbon atoms or a substituted 1,2-diaminocycloalkane;
wherein L is a purine or pyrimidine nucleoside or nucleotide or a purine or pyrimidine nucleoside or nucleotide which is substituted on the purine or pyrimidine nucleus;
wherein X is a monodentate anionic ligand; wherein Y is an anion and wherein n = 0 or 1.

2. The composition of claim 1, wherein the monodentate amine is $R^1NH_2$, wherein $R^1$ is H or a linear or branched $C_1$-$C_6$ alkyl group or a substituted linear or branched $C_1$-$C_6$ alkyl group.

3. The composition of claim 1 or 2, wherein the substituted linear or branched $C_1$-$C_6$ alkyl groups and the substituted 1,2-diaminocycloalkane are substituted with one or more substitutents selected from the group consisting of hydroxy, $C_1$-$C_6$ alkyl, $C_1$-$C_4$ alkoxy, halo, carboxy, $C_1$-$C_4$ alkylcarboxy and $C_1$-$C_4$ alkoxycarbonyl; wherein the subtituted purine or pyrimidine is substituted by one or more substituents selected from the group consisting of $C_1$-$C_6$ alkyl, $C_1$-$C_6$ alkoxy, hydroxy, carboxy, $C_1$-$C_6$ alkoxycarbonyl, $C_1$-$C_6$ alkylcarboxy, amino, oxo, nitro or amido; wherein X is selected from the group consisting of halo, hydroxo, pseudohalo, $HSO_4$, $H_2PO_4$, ascorbate and $C_1$-$C_6$ carboxylate; and wherein Y is selected from the group consisting of halo, hydroxo, pseudohalo, $HSO_4$, $H_2PO_4$, nitrate, perchlorate, ascorbate and $C_1$-$C_6$ carboxylate.

4. The composition of claim 1, 2 or 3, wherein L is selected from the group consisting of guanosine, 2′-deoxyguanosine, cytidine, 2′-deoxycytidine and 1-β-D-arabinofuranosylcytosine.

5. The composition of claim 1, which contains a compound selected from cis-[Pt($NH_3$)$_2$(N3-cytidine)Cl]$NO_3$,
cis-[Pt($NH_3$)$_2$(N7-guanosine)Cl]$NO_3$,
cis-[Pt($NH_3$)$_2$(N3-2′deoxycytidine)Cl]Cl,
cis-[Pt($NH_3$)$_2$(N3-1-β-D-arabinofuranosylcytosine)Cl]$NO_3$, and
cis-[Pt($NH_3$)$_2$(N7-2′-deoxyguanosine)Cl]$NO_3$

6. The composition of any of claims 1 to 5, in a form suitable for parenteral administration.

7. The composition of any of claims 1 to 5, in a form suitable for oral administration.

8. The composition of any of claim 1 to 5, in tablet form.

9. The composition of any of claims 1 to 5, in capsule form.

10. A method of making a composition according to any of claims 1 to 9, comprising mixing the said active ingredient with the said diluent or carrier.

11. A method of treating malignant animal tumors sensitive to a compound of the general formula I as defined in any of claims 1 to 5, comprising administering the compound or a mixture of such compounds to an animal afflicted with such a tumor in an amount sufficient to cause regression of the tumor cells.

12. A compound of the general formula I as defined in any of claims 1 to 5, for use in the treatment of the human or animal body by therapy.

13. The use of a compound of the general formula I as defined in any of claims 1 to 5 in the manufacture of a medicament for treating animal malignant tumor cells.